# EUROPÄISCHE PATENTSCHRIFT

(11) **EP 3 784 656 B1**
(45) Veröffentlichungstag und Bekanntmachung des Hinweises auf die Patenterteilung: **28.06.2023**
(21) Anmeldenummer: 19720502.4
(22) Anmeldetag: 18.04.2019
(51) Int. Cl.: C07D 215/56

(54) **VERFAHREN ZUR HYDROLYSE VON CHINOLONCARBONSÄUREESTERN**
PROCESS FOR THE HYDROLYSIS OF QUINOLONE CARBOXYLIC ESTERS
PROCÉDÉ D'HYDROLYSE D'ESTERS D'ACIDES QUINOLONE-CARBOXYLIQUES

(30) Priorität: 25.04.2018 EP 18169170
(43) Veröffentlichungstag der Anmeldung: 03.03.2021
(73) Patentinhaber: Elanco Animal Health GmbH, 40789 Monheim am Rhein (DE)
(72) Erfinder: FEY, Peter, 42111 Wuppertal (DE); BERWE, Mathias, 45549 Sprockhövel (DE); WIRTHS, Jörg, 45289 Essen (DE); WISCHNAT, Ralf, 51467 Bergisch Gladbach (DE); LONGERICH, Markus, 50825 Köln (DE); DIETZEL, Antje, 45127 Essen (DE)
(74) Vertreter: Cohausz & Florack
(86) Internationale Anmeldenummer: PCT/EP2019/060072
(87) Internationale Veröffentlichungsnummer: WO 2019/206798

(56) Entgegenhaltungen:
- EP-A1- 0 276 700
- EP-A1- 1 236 718
- EP-A1- 1 319 656
- WO-A1-98/26779
- WO-A2-2004/014893

## Beschreibung

Die vorliegende Erfindung betrifft ein Verfahren zur Hydrolyse von Chinoloncarbonsäureestern zu Chinoloncarbonsäuren. Fluorchinoloncarbonsäuren sind wichtige Zwischenprodukte zur Herstellung von bekannten pharmazeutisch wirksamen Verbindungen aus der Klasse der Chinolone. Als konkrete Beispiele seien genannt: Benofloxacin, Binfloxacin, Cinoxacin, Ciprofloxacin, Danofloxacin, Difloxacin, Enoxacin, Enrofloxacin, Fleroxacin, Ibafloxacin, Levofloxacin, Lomefloxacin, Marbofloxacin, Moxifloxacin, Norfloxacin, Ofloxacin, Orbifloxacin, Pefloxacin, Pipemidsäure, Temafloxacin, Tosufloxacin, Sarafloxacin, Sparfloxacin und Pradofloxacin.

Pradofloxacin ist ein hoch wirksames Chinolonantibiotikum in der Veterinärmedizin. Seine antibakterielle Wirkung sowie Indikationen, Anwendungsformen und geeignete Zubereitungen sind beispielsweise in WO 97/31001 A1, WO 03/007995 A1, WO 03/101422 A1, WO 04/082658 A1, WO 05/018641 A1, WO 05/044271 A1 und WO 06/061156 A1.

Ein Schritt in der mehrstufigen Synthese von Pradofloxacin ist die Hydrolyse eines Chinoloncarbonsäureethylesters. Die Senkung des pH-Wertes für die Hydrolyse von Chinoloncarbonsäureestern kann mittels Salzsäure oder Schwefelsäure/Essigsäure erfolgen. Das Salzsäureverfahren hat den Nachteil, dass das Reaktionsgemisch sehr korrosiv ist und die für die Durchführung der Reaktion vorgesehene Anlage entsprechend korrosionsfest sein muss. Dieses hat hohe Kosten zur Folge. Ferner müssen die Mutterlaugen vor der Entsorgung unter Kosteneinsatz neutralisiert werden, es fallen große Abfallmengen an und der Prozess hat eine vergleichsweise hohe Anzahl an Schritten.

Das Schwefelsäure/Essigsäureverfahren hat den Vorteil, dass die eingesetzte Essigsäure durch Destillation wiedergewonnen werden kann und dass weniger korrosive Medien eingesetzt werden können.

WO 98/26779 A1offenbart in Beispiel Z 22 der Beschreibung die Synthese von 7-Chlor-8-cyano-1-cyclopropyl-6-fluor-1,4-dihydro-4-oxo-3-chinolincarbonsäure. Hierzu wurden 3. 8 g (0.1 mol) 7-Chlor-8-cyano-1-cyclopropyl-6-fluor-1,4-dihydro-4-oxo-3-chinolincarbonsäureethylester in einer Mischung aus 100 ml Essigsäure, 20 ml Wasser und 10 ml konzentrierter Schwefelsäure für 3 Stunden unter Rückfluss erhitzt. Nach dem Abkühlen wurde auf 100 ml Eiswasser ausgegossen, der ausgefallene Niederschlag abgesaugt, mit Wasser und Ethanol gewaschen und bei 60 °C im Vakuum getrocknet. Pro mol des Esters wurden 17.3 mol Essigsäure, 1.86 mol Schwefelsäure und 11 mol Wasser eingesetzt.

EP 0 276 700 A1 offenbart in Beispiel 1 der Beschreibung die Hydrolyse von 7-Chlor-8-cyano-1-cyclopropyl-6-fluor-1,4-dihydro-4-oxo-3-chinolincarbonsäureethylester. 1 g dieser Verbindung wurden zusammen mit 3.5 ml Essigsäure, 3 ml Wasser und 0.3 ml Schwefelsäure für 4 Stunden auf 140 -145 °C erhitzt. Anschließend wurde mit Wasser verdünnt und der Feststoff isoliert. Es wurden 0.7 g der freien Carbonsäure vom Schmelzpunkt 281 - 282 °C erhalten. Pro mol des Esters wurden 20 mol Essigsäure, 1.89 mol Schwefelsäure und 55.8 mol Wasser eingesetzt.

EP 0 169 993 A2 offenbart in Beispiel A der Beschreibung, dass ein Gemisch von 94 g 1-Cyclopropyl-6,7,8-trifluor-1,4-dihydro-4-oxo-3-chinolincarbonsäureethylester, 600 ml Eisessig, 450 ml Wasser und 70 ml konzentrierter Schwefelsäure für 1.5 Stunden auf Rückfluss erhitzt wurde. Dann goss man die heiße Suspension auf Eis, saugte den Niederschlag ab, wusch mit Wasser nach und trocknete im Vakuum bei 100 °C. Man erhielt auf diese Weise 88.9 g 1-Cyclopropyl-6,7,8-trifluor-1,4-dihydro-4-oxo-3-chinolincarbonsäure. Pro mol des Esters wurden 34.7 mol Essigsäure, 4.35 mol Schwefelsäure und 82.8 mol Wasser eingesetzt.

EP 1 319 656 A1 offenbart in Beispiel 2 die Reaktion von 29.4 g Ethyl-1-cyclopropyl-7-chlor-6-fluor-8-methoxy-1,4-dihydro-4-oxo-3-chinolincarboxylat (0.088 mol), 160 ml Essigsäure, 100 ml Wasser und 18 ml konzentrierter Schwefelsäure. Man rührte bei 100 - 110 °C für 40 Minuten. Die erhaltene Mischung wurde abgekühlt und filtriert. Der Niederschlag wurde aus Chloroform-Ethanol umkristallisiert. Man erhielt 23.8 g der freien Carbonsäure. Pro mol des Esters wurden 31.8 mol Essigsäure, 3.84 mol Schwefelsäure und 61.1 mol Wasser eingesetzt.

EP 1 236 718 A1 offenbart in Beispiel 1, dass 300 g 1-Cyclopropyl-6,7,8-trifluor-1,4-dihydro-4-oxo-3-chinolin-carbonsäureethylester, 106.8 g Wasser und 426 g Essigsäure vorgelegt wurden und 3.8 g Schwefelsäure zugesetzt wurden. Die Mischung wurde 3 Stunden auf Rückfluss erhitzt. Anschliessend wurden bis zum Erreichen einer Sumpftemperatur von 109 °CC 310 ml Destillat abdestilliert. Dann wurde das Gemisch auf 80 °C abgekühlt und 157.5 g 4,8 gew.-%ige Natriumacetatlösung zugetropft. Der pH-Wert lag dann im Bereich 3 bis 4. Das Gemisch wurde anschliessend auf 20 °C abgekühlt und der Feststoff abgesaugt. Der Feststoff wurde mit 200 ml Wasser gewaschen und im Vakuum bei 50 °C getrocknet. Es wurden 270.3 g 1-Cyclopropyl-6,7,8-trifluor-1,4-dihydro-4-oxo-3-chinolin-carbonsäure isoliert, was einer Ausbeute von 99 % der Theorie entspricht. Pro mol des Esters wurden 7.4 mol Essigsäure, 0.04 mol Schwefelsäure und 6.2 mol Wasser eingesetzt.

Die vorliegende Erfindung hat sich die Aufgabe gestellt, ein verbessertes Verfahren zur Hydrolyse von Chinoloncarbonsäureestern bereitzustellen, bei dem möglichst wenig aufzubereitende Abfallprodukte entstehen und bei dem die erhaltene Chinoloncarbonsäure eine möglichst hohe Reinheit aufweist.

Erfindungsgemäß gelöst wird die Aufgabe durch ein Verfahren zur Hydrolyse von Chinoloncarbonsäureestern der allgemeinen Formel (II) unter Erhalt von Chinoloncarbonsäuren der allgemeinen Formel (I): 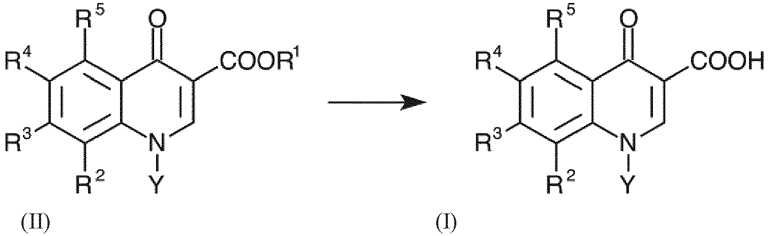
wobei in Formel (II) R¹ für Methyl oder Ethyl steht und in Formeln (I) und (II) übereinstimmend:
   R² für Wasserstoff, Methyl, Methoxy, Fluor, Chlor, Nitro oder Cyano steht,
   R³ für Fluor oder Chlor steht und R² für Fluor steht,
   R⁵ für Wasserstoff, Methyl, Fluor, Chlor oder Nitro steht, und
   Y für Methyl, Ethyl, Isopropyl, Cyclopropyl, Fluorcyclopropyl, 4-Fluorphenyl oder 2,4-Difluorphenyl steht umfassend den Schritt:
      A) Reaktion von Verbindungen der Formel (II) mit einer Mischung umfassend Essigsäure, Schwefelsäure und Wasser.

In Schritt A) werden pro mol Verbindungen der Formel (II) ≥ 30 bis ≤ 40 mol Essigsäure, >_ 0.3 bis ≤ 1 mol Schwefelsäure und ≥ 0,9 bis ≤ 2.5 mol Wasser eingesetzt. Vorzugsweise werden pro mol Verbindungen der Formel (II) ≥ 32 bis ≤ 38 mol Essigsäure, >_ 0.4 bis ≤ 0.8 mol Schwefelsäure und ≥ 0.9 bis ≤ 2.3 mol Wasser, mehr bevorzugt ≥ 33 bis ≤ 35 mol Essigsäure, ≥ 0.4 bis ≤ 0.6 mol Schwefelsäure und ≥ 0.9 bis ≤ 2.2 mol Wasser eingesetzt.

In dem erfindungsgemäßen Verfahren kann man Essigsäure und Schwefelsäure in wasserhaltiger oder wasserfreier Form einsetzen. Die beschriebenen Mengenangaben beziehen sich auf 100 %ige Essigsäure und 100 %ige Schwefelsäure. Wenn man Wasser enthaltende Essigsäure und/oder Wasser enthaltende Schwefelsäure einsetzt, muss man entsprechend deren Wassergehalt weniger Wasser einsetzen. Essigsäure wird vorzugsweise in Form von Eisessig, Schwefelsäure vorzugsweise in Form von 96 bis 100 %iger Schwefelsäure eingesetzt.

Die Zugabe von Wasser, Essigsäure und Schwefelsäure erfolgt vorzugsweise so, dass man den Ester (II), die Essigsäure und die Schwefelsäure vorlegt und anschließend das Wasser zufügt. Es ist ebenfalls möglich, zuerst den Ester (II), das Wasser und die Essigsäure vorzulegen und anschließend die Schwefelsäure hinzuzufügen. Vorzugsweise wird das Reaktionsgemisch für 10 bis 25 Stunden, mehr bevorzugt 12 bis 22, besonders bevorzugt 16 bis 20 Stunden, erhitzt.

Vorzugsweise werden in Schritt A) neben dem Ester (II), Essigsäure, Wasser und Schwefelsäure keine weiteren chemisch reaktiven oder katalytisch aktiven Verbindungen eingesetzt.

Bevorzugte Ausführungsformen des erfindungsgemäßen Verfahrens werden nachfolgend beschrieben. Sie können beliebig miteinander kombiniert werden, sofern sich aus dem Kontext nicht eindeutig das Gegenteil ergibt.

In einer Ausführungsform des Verfahrens werden in Schritt A) ≥ 95 mol-% der eingesetzten Verbindungen der Formel (II) zu Verbindungen der Formel (I) umgesetzt. Vorzugsweise beträgt diese Ausbeute ≥ 96 mol-%, mehr bevorzugt ≥ 99.5 mol-%.

In einer weiteren Ausführungsform des Verfahrens wird die Reaktion in Schritt A) bei einer Temperatur von ≥ 90 bis ≤ 99 °C durchgeführt. Vorzugsweise beträgt diese Temperatur ≥ 92 bis ≤ 97 °C, mehr bevorzugt >_ 94 bis ≤ 95 °C. Es hat sich herausgestellt, dass höhere Reaktionstemperaturen in diesem Schritt zu einem erhöhten Gehalt an Verunreinigungen führen (vgl. die weiter hinten angegebenen analytischen Daten zu Beispielen und Vergleichsbeispielen)

Das Erhitzen des Reaktionsgemisches kann bei vermindertem Druck, atmosphärischem Druck oder erhöhtem Druck durchgeführt werden. Beispielsweise sind Drücke im Bereich von 0,5 bis 3 bar möglich. Sofern nicht anders angegeben arbeitet man üblicherweise in allen hier beschriebenen Verfahrensschritten bei atmosphärischem Druck.

Ein Vorteil des erfindungsgemäßen Verfahrens ist, dass keine Destillation erforderlich ist und dass das Produkt direkt aus dem Reaktionsgemisch durch Filtration isoliert werden kann. Dies ist kostengünstiger als die Vergleichsverfahren mit Destillationsschritt.

Die Isolierung der hergestellten Chinoloncarbonsäure aus vorliegenden Gemisch kann beispielsweise so erfolgen, dass man den dann vorliegenden Niederschlag absaugt, wäscht und und trocknet. Zum Waschen des Niederschlags wird vorzugsweise Ethanol verwendet, besonders bevorzugt wird erst mit Essigsäure und dann mit Ethanol gewaschen. Ein Waschen mit Wasser kann vermieden werden, wodurch auch die aufgefangene Essigsäure leichter wiedergewonnen werden kann. Es ist vorteilhaft, das isolierte Produkt mehrmals zu waschen, um es hinreichend frei und weitgehend ohne anhaftende Schwefelsäure zu erhalten. Ein Zusatz von Base ist im erfindungsgemäßen Verfahren an dieser Stelle nicht erforderlich. Dieses spart auch wieder Abfälle und Kosten.

Außerhalb des Schutzumfangs des Anspruchs 1 liegt ein Verfahren für das gilt dass in Formeln (I) und (II) übereinstimmend:
R² für Wasserstoff, Methyl, Methoxy, Fluor, Chlor, Nitro oder Cyano steht,
R³ für Fluor oder Chlor steht,
R⁴ für Fluor steht,
R⁵ für Wasserstoff, Methyl, Fluor, Chlor oder Nitro steht,
Y für Methyl, Ethyl, Isopropyl, Cyclopropyl, Fluorcyclopropyl, 4-Fluorphenyl oder 2,4-Difluorphenyl steht
und in Formel (I) R¹ für Methyl oder Ethyl steht.

Ebenso ist es möglich, dass in Formeln (I) und (II) übereinstimmend:
R² für Wasserstoff, C1-C4-Alkoxy oder Cyano steht,
R³ für Halogen, insbesondere Chlor, steht,
R⁴ für Fluor steht,
R⁵ für Wasserstoff steht,
Y für Cyclopropyl steht
und in Formel (I) R¹ für Methyl oder Ethyl steht.

In einer weiteren Ausführungsform des Verfahrens steht Formel (I) für 1-Cyclopropyl-6,7,8-trifluor-1,4-dihydro-4-oxo-3-chinolin-carbonsäure, 1-Cyclopropyl-6,7-difluor-1,4-dihydro-4-oxo-3-chinolin-carbonsäure, 1 -Cyclopropyl-6,7-difluor-8-cyano-1,4-dihydro-4-oxo-3-chinolin-carbonsäure, 1-(2-Fluor)cyclopropyl-6,7-difluor-1,4-dihydro-4-oxo-3 -chinolin-carbonsäure, 1-Cyclopropyl-8-chlor-6,7-difluor-1,4-dihydro-4-oxo-3-chinolin-carbonsäure, 1-Ethyl-6,7,8-trifluor-1,4-dihydro-4-oxo-3-chinolin-carbonsäure oder 1-Cyclopropyl-6-fluor-7-chlor-8-cyano-1,4-dihydro-4-oxo-3-chinolin-carbonsäure.

In einer weiteren Ausführungsform des Verfahrens haben Formeln (II) und (I) die folgende Bedeutung gemäß den Formeln (II-1) bzw. (I-1):

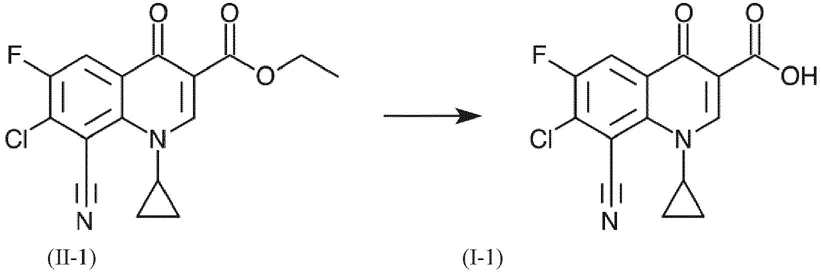

In einer weiteren Ausführungsform des Verfahrens sind die Verbindungen der Formel (II) durch Reaktion von Verbindungen der allgemeinen Formel (III) erhältlich: 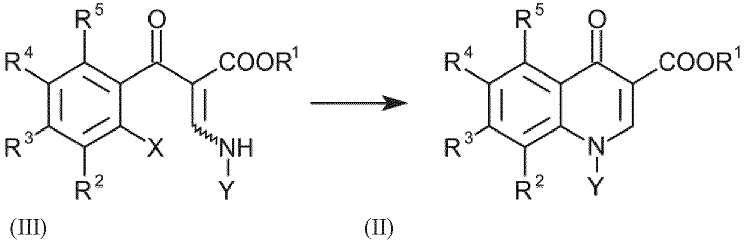 wobei R¹ bis R⁵ und Y die vorgenannten Bedeutungen haben und X für Halogen steht.

In einer weiteren Ausführungsform des Verfahrens sind die Verbindungen der Formel (III) durch Reaktion von Verbindungen der allgemeinen Formel (IV) erhältlich: 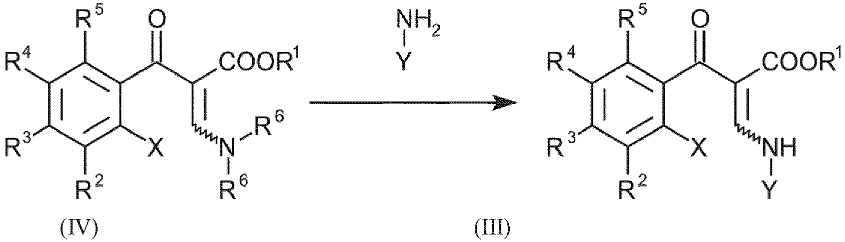 und wobei R¹ bis R⁵, X und Y die vorgenannten Bedeutungen haben und R⁶ für C₁-C₄-Alkyl steht.

In einer weiteren Ausführungsform des Verfahrens sind die Verbindungen der Formel (IV) durch Reaktion von Verbindungen der allgemeinen Formel (V) erhältlich: 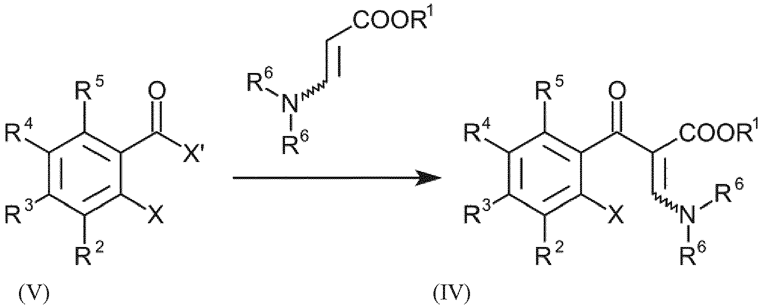 und wobei R¹ bis R⁶, X und Y die vorgenannten Bedeutungen haben und X' für Halogen steht.

Für die bevorzugte Herstellung der Pradofloxacin-Zwischenstufe (I) wäre in der Reaktionssequenz:
(V) → (IV) → (III) → (II) → (I)
in allen betreffenden Verbindungen dieser allgemeinen Formeln R¹ Ethyl, R² Cyano, R³ Chlor, R⁴ Fluor, R⁵ Wasserstoff, R⁶ Methyl, X Chlor, X' Chlor und Y Cyclopropyl.

### Beispiele

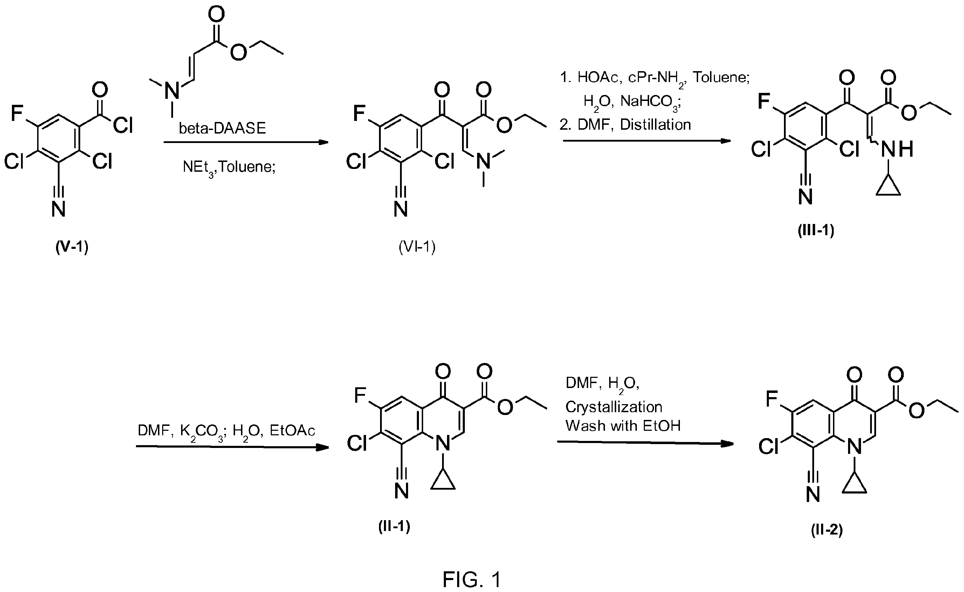

Die vorliegende Erfindung wird durch die nachfolgenden Beispiele näher erläutert, ohne jedoch darauf beschränkt zu sein. Ein Syntheseschema ist in FIG 1 wiedergegeben.

### Beispiel 1

### Synthese von Ethyl-7-chlor-8-cyan-1-cyclopropyl-6-fluor-4-oxo-1,4-dihydrochinolin-3-carboxylat (Formel (II-2) in FIG. 1)

Zu 11.8 kg Toluol wurden 2.70 kg (18.8 mol) Ethyl-(2E)-3-(dimethylamino)acrylat (beta-Dimethylaminoacrylat, β-DAASE) und 2.12 kg (20.9 mol) Triethylamin gegeben auf Ti = 45 °C bis 55 °C erwärmt. Danach wurde eine Lösung aus 4.50 kg (17.8 mol) 2,4-Dichlor-3-cyan-5-fluorbenzoylchlorid (Formel (V-1) in FIG. 1) in 11.6 kg Toluol bei Ti = 50 °C zudosiert. Es wurde 3 Stunden bei Ti = 50 °C gerührt und auf Ti = 22 °C abgekühlt. Die Suspension wurde filtriert und der Filterkuchen mit 3.4 kg Toluol gewaschen. Zum Filtrat (Formel (IV-1) in FIG 1) wurden 1.23 kg (20.5 mol) Essigsäure und eine Lösung aus 1.11 kg (19.4 mol) Cyclopropylamin in 2.40 kg Toluol innerhalb von 2 Stunden bei Ti = 5-15 °C zudosiert und 5 Stunden bei Ti = 10 °C nachgerührt. Anschließend wurden 13.5 kg Wasser zugegeben, das Gemisch auf Ti = 40 °C erwärmt und 30 Minuten bei dieser Temperatur nachgerührt. Danach wurden die Phasen bei Ti = 40 °C getrennt. Es wurde eine Lösung aus 300 g Natriumcarbonat in 6.0 kg Wasser zur organischen Phase gegeben, das Gemisch wurde auf Ti = 40 °C erwärmt, 30 Minuten nachgerührt und die Phasen bei Ti = 40 °C getrennt.

Von der organischen Phase wurde im Vakuum bis zu eine Manteltemperatur von 60 °C eine Menge von 22.6 L abdestilliert. Danach wurden 19.2 kg N,N-Dimethylformamid zugegeben und für mindestens10 Minuten bei 40 °C gerührt. Danach wurde erneut im Vakuum bis zu einer Manteltemperatur von Tm = 60 °C destilliert bis kein Destillat mehr überging und der Rückstand (Formel (III-1) in FIG. 1) auf Raumtemperatur abgekühlt.

Zum Rückstand wurden 2.22 kg (16.0 mol) Pottasche gegeben, die Suspension auf Ti = 55 °C erwärmt und 5 h bei dieser Temperatur gerührt. Es wurde auf Ti = 22 °C abgekühlt und im Vakuum bis zu einer Manteltemperatur von 80 °C eine Destillatmenge von 16.5 l abdestilliert. Zu dem Rückstand wurden 18.0 kg Wasser gegeben und bei Ti = 55 °C mindestens 10 Minuten nachgerührt. Danach wurde innerhalb von 2 Stunden auf Ti = 5 °C abgekühlt und weitere 2 Stunden bei dieser Temperatur gerührt.

Das erhaltene Produkt wurde abfiltriert, zweimal mit je 6.0 kg Wasser gewaschen und mindestens 3 Stunden bei Ti = 22 °C mit 9.9 kg Ethylacetat ausgerührt. Die Suspension wurde filtriert und der Filterkuchen noch zweimal mit je 4.8 kg Ethylacetat gewaschen und mindestens 12 Stunden bei 50 °C im Vakuum Rohprodukt (Formel (II-1) in FIG 1) getrocknet.

Ausbeute: 5.08 kg; 85.1% d.Th. bezogen auf 2,4-Dichlor-3-cyan-5-fluor-benzoylchlorid.

2.96 kg Rohprodukt wurden in 29.4 kg N,N- Dimethylformamid auf 60 °C erwärmt und bei dieser Temperatur unlösliche Verunreinigungen abfiltriert. Zum Filtrat wurden 3.8 kg-Wasser gegeben es wurde 1.5 Stunden nachgerührt und anschließend 13.9 kg Wasser in 1.5 Stunden zudosiert. Die erhaltene Suspension wurde auf Ti = 22 °C abgekühlt, 30 Minuten nachgerührt und der Feststoff abfiltriert. Der Nutschkuchen wurde mit 3.1 kg Wasser, anschließend zweimal mit je 2.8 kg Ethanol gewaschen und bei 50 °C im Vakuum mindestens 12 Stunden getrocknet (Formel (II-2) in FIG 1). Das hier beschriebene Verfahren hat den Vorteil, dass ein Ester (II-2) in hoher Reinheit hergestellt werden kann. Dieses ist günstig für die Herstellung der freien Chinoloncarbonsäuren in hoher Reinheit. (Der Ester der Formel (II-2) wird durch einen Reinigungsschritt aus dem Ester der Formel (II-1) hergestellt. Beide Formeln beschreiben daher dieselbe chemische Struktur. Die verschiedene Bezeichnung der Formeln soll nur den unterschiedlichen Reinheitsgrad verdeutlichen)

Ausbeute: 2.87 kg; ca.97% d.Th. bezogen auf Rohprodukt

### Beispiel 2

### Hydrolyse des Produkts aus Beispiel 1

Zu 20.0 g (59.8 mmol) des Produkts aus Beispiel 1 (Formel (II-2) in FIG 1) wurden 122.8 g (2.04 mol) Essigsäure, 3.37 g (33.0 mmol) Schwefelsäure und 1.1 g (63.0 mmol) Wasser gegeben. Es wurde auf 95 °C erhitzt und 18.5 h bei dieser Temperatur gerührt. Die Suspension wurde auf 10 °C gekühlt, der Feststoff wurde abgesaugt, mit 48 ml Essigsäure und anschließend mit 48 ml Ethanol gewaschen und über Nacht bei 60 °C im Vakuumtrockenschrank getrocknet.

Pro mol (II-2) wurden eingesetzt: 34.11 mol Essigsäure, 0.55 mol Schwefelsäure und 1.05 mol Wasser.

Ausbeute: 17.6 g; 96.1% d. Th.

Zur Reinheit siehe Tabelle weiter unten.

### Beispiel 3

### Hydrolyse des Produkts aus Beispiel 1

Zu 20.0 g (59.8 mmol) des Produkts aus Beispiel 1 (Formel (II-2) in FIG 1) wurden 122.8 g (2.04 mol) Essigsäure, 3.37 g (33.0 mmol) Schwefelsäure und 2.3 g (126.0 mmol) Wasser gegeben. Es wurde auf 95 °C erhitzt und 18.5 h bei dieser Temperatur gerührt. Die Suspension wurde auf 10 °C gekühlt, der Feststoff wurde abgesaugt, mit 48 ml Essigsäure und anschließend mit 48 ml Ethanol gewaschen und über Nacht bei 60 °C im Vakuumtrockenschrank getrocknet.

Pro mol (II-2) wurden eingesetzt: 34.11 mol Essigsäure, 0.55 mol Schwefelsäure und 2.10 mol Wasser.

Ausbeute: 17.8 g; 97.1% d. Th.

Zur Reinheit siehe Tabelle weiter unten.

### Vergleichsbeispiel 1

### nach Vorschrift Beispiel 1 aus EP1236718

Zu 30.0 g (89.6 mmol) des Produkts aus Beispiel 1 (Formel (II-2) in FIG 1) wurden 39.6 g (656.1 mmol) Essigsäure, 0.20 ml (3.5 mmol) Schwefelsäure und 9.9 g (549.5 mmol) Wasser gegeben. Es wurde 3 h unter Rückfluß erhitzt. Anschließend wurde bis zum Erreichen einer Sumpftemperatur von 109 °C 14.1 g Destillat abdestilliert. Das Gemisch wurde auf 80 °C abgekühlt und 40.1 g 4.8 Gew.-%ige Natriumacetatlösung zugetropft. Der pH-Wert lag dann im Bereich 3 bis 4. Das Gemisch wurde anschließend auf 20 °C abgekühlt und der Feststoff abgesaugt. Der Feststoff wurde mit 50 ml Wasser gewaschen und im Vakuum bei 50 °C getrocknet.

Pro mol (II-2) wurden eingesetzt: 7.32 mol Essigsäure, 0.04 mol Schwefelsäure und 6.13 mol Wasser.

Ausbeute: 26.7 g; 97.2% d. Th.

Zur Reinheit siehe Tabelle weiter unten.

### Vergleichsbeispiel 2

### nach Vorschrift Beispiel 2 aus EP 1 236 718

Zu 30,0 g (89.6 mmol) des Produkts aus Beispiel 1 (Formel (II-2) in FIG 1) wurden 78.9 g (1.31 mol) Essigsäure, 0.51 ml (9.1 mmol) Schwefelsäure und 2.25 g (124.9 mmol) Wasser gegeben. Es wurde 4 h unter Rückfluß erhitzt. Anschließend wurde bis zum Erreichen einer Sumpftemperatur von 109 °C 8.1 g Destillat abdestilliert. Das Gemisch wurde auf 80 °C abgekühlt und 75.3 g 4.8 Gew.-%ige Natriumacetatlösung zugetropft. Der pH-Wert lag dann im Bereich 3 bis 4. Das Gemisch wurde anschließend auf 20 °C abgekühlt und der Feststoff abgesaugt. Der Feststoff wurde mit 50 ml Wasser gewaschen und im Vakuum bei 50 °C getrocknet.

Pro mol (II-2) wurden eingesetzt: 14.62 mol Essigsäure, 0.10 mol Schwefelsäure und 1.39 mol Wasser.

Ausbeute: 27.0 g; 98.2% d. Th.

Zur Reinheit siehe Tabelle weiter unten.

### Vergleichsbeispiel 3

### nach Vorschrift Beispiel Z 22 aus WO98/26779

Zu 9.5 g (28.4 mmol) des Produkts aus Beispiel 1 (Formel (II-2) in FIG. 1) wurden 29.5 g (490.9 mmol) Essigsäure, 5.2 g (52.5 mmol) Schwefelsäure und 5.6 g (310.2 mmol) Wasser gegeben. Es wurde 3 h unter Rückfluß erhitzt und auf 20 °C abgekühlt. Anschließend wurde das Gemisch auf 28g Eiswasser gegeben und der Feststoff abgesaugt. Der Feststoff wurde mit 100 ml Wasser gewaschen und 10 ml Ethanol und im Vakuum bei 60 °C getrocknet.

Pro mol (II-2) wurden eingesetzt: 17.28 mol Essigsäure, 1.85 mol Schwefelsäure und 10.92 mol Wasser.

Ausbeute: 8.5 g; 97.7% d. Th.

Die nachfolgende Tabelle fasst die Reinheiten der in den Beispielen und Vergleichsbeispielen erhaltenen Hydrolyseprodukte der Formel I-1 zusammen:

| | Beispiel 2 | Beispiel 3 | Vergleich 1 | Vergleich 2 | Vergleich 3 |
|---|---|---|---|---|---|
| Restgehalt an Ethylester (II-2)^{∗} | 0.196 | 0.268 | 0.772 | 0.436 | 0.108 |
| Summe Gehalt an unspezifizierten Verunreinigungen^{∗} | 0.005 | 0.007 | 0.124 | 0.130 | 0.146 |
| Assay i.d.s.^{∗∗} | 100.116 | 100.349 | 97.904 | 97.398 | 99,6 |

| | | | | | |
|---|---|---|---|---|---|
| ^{∗} Angaben in Flächenprozent (bestimmt durch HPLC-Analyse) ^{∗∗} Angaben in Gewichtsprozent i.d.s bedeutet "in dried substance" (in getrockneter Substanz) | | | | | |

Man erkennt, dass im erfindungsgemäßen Verfahren Produkte mit größerer Reinheit als in den Vergleichsverfahren erhalten werden, in denen nicht die erfindungsgemäß geforderten Verhältnisse von Edukt (II-2), Essigsäure, Schwefelsäure und Wasser eingehalten werden.

## Patentansprüche

1. Verfahren zur Hydrolyse von Chinoloncarbonsäureestern der allgemeinen Formel (II) unter Erhalt von Chinoloncarbonsäuren der allgemeinen Formel (I): 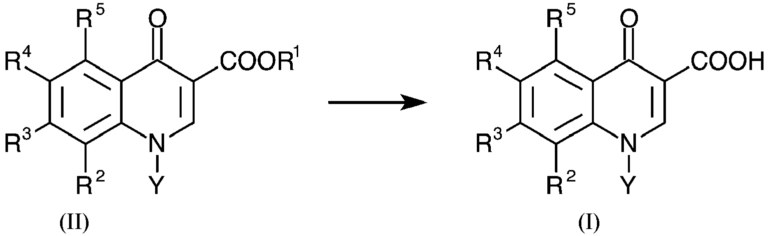
wobei in Formeln (I) und (II) übereinstimmend
R² für Wasserstoff, Methyl, Methoxy, Fluor, Chlor, Nitro oder Cyano steht,
R³ für Fluor oder Chlor steht,
R⁴ für Fluor steht,
R⁵ für Wasserstoff, Methyl, Fluor, Chlor oder Nitro steht,
Y für Methyl, Ethyl, Isopropyl, Cyclopropyl, Fluorcyclopropyl, 4-Fluorphenyl oder 2,4-Difluorphenyl steht
und in Formel (I) R¹ für Methyl oder Ethyl steht,
umfassend den Schritt:
A) Reaktion von Verbindungen der Formel (II) mit einer Mischung umfassend Essigsäure, Schwefelsäure und Wasser
**dadurch gekennzeichnet, dass**
in Schritt A) pro mol Verbindungen der Formel (II) ≥ 30 bis ≤ 40 mol Essigsäure, ≥ 0.3 bis ≤ 1 mol Schwefelsäure und ≥ 0.9 bis ≤ 2.5 mol Wasser eingesetzt werden.

2. Verfahren gemäß Anspruch 1, wobei in Schritt A) ≥ 95 mol-% der eingesetzten Verbindungen der Formel (II) zu Verbindungen der Formel (I) umgesetzt werden.

3. Verfahren gemäß Anspruch 1 oder 2, wobei die Reaktion in Schritt A) bei einer Temperatur von ≥ 90 bis ≤ 99 °C durchgeführt wird.

4. Verfahren gemäß einem der Ansprüche 1 bis 3, wobei Formel (I) für 1-Cyclopropyl-6,7,8-trifluor-1,4-dihydro-4-oxo-3-chinolin-carbonsäure, 1-Cyclopropyl-6,7-difluor-1,4-dihydro-4-oxo-3-chinolin-carbonsäure, 1-Cyclopropyl-6,7-difluor-8-cyano-1,4-dihydro-4-oxo-3-chinolin-carbonsäure, 1-(2-Fluor)cyclopropyl-6,7-difluor-1,4-dihydro-4-oxo-3-chinolin-carbonsäure, 1-Cyclopropyl-8-chlor-6,7-difluor-1,4-dihydro-4-oxo-3-chinolin-carbonsäure, 1-Ethyl-6,7,8-trifluor-1,4-dihydro-4-oxo-3-chinolin-carbonsäure oder 1-Cyclopropyl-6-fluor-7-chlor-8-cyano-1,4-dihydro-4-oxo-3-chinolin-carbonsäure steht.

5. Verfahren gemäß einem der Ansprüche 1 bis 4, wobei Formeln (II) und (I) die folgende Bedeutung gemäß den Formeln (II-1) bzw. (1-1) haben:

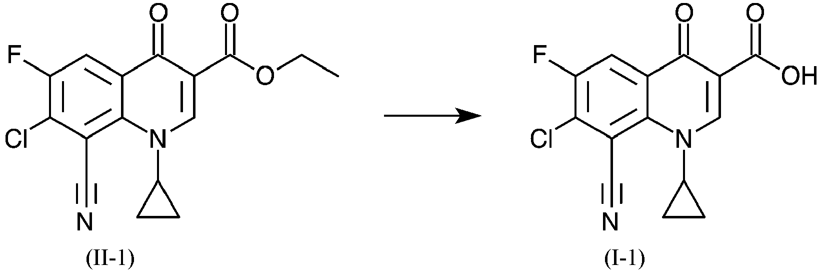

6. Verfahren gemäß einem der Ansprüche 1 bis 5, wobei die Verbindungen der Formel (II) durch Reaktion von Verbindungen der allgemeinen Formel (III) erhältlich sind: 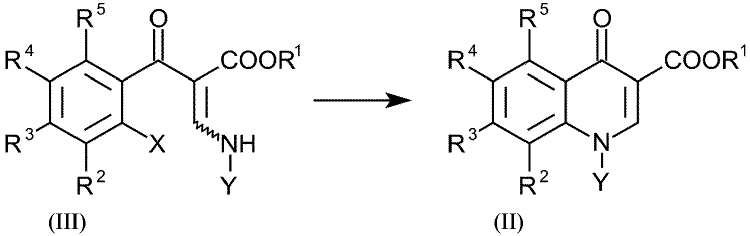 wobei R¹ bis R⁵ und Y die vorgenannten Bedeutungen haben und X für Halogen steht.

7. Verfahren gemäß Anspruch 6, wobei die Verbindungen der Formel (III) durch Reaktion von Verbindungen der allgemeinen Formel (IV) erhältlich sind: 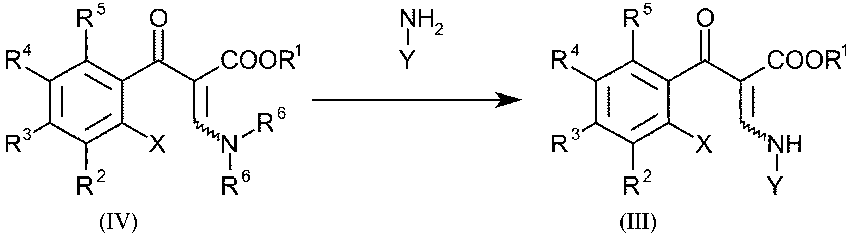 und wobei R¹ bis R⁵, X und Y die vorgenannten Bedeutungen haben und R⁶ für C₁-C₄-Alkyl steht.

8. Verfahren gemäß Anspruch 7, wobei die Verbindungen der Formel (IV) durch Reaktion von Verbindungen der allgemeinen Formel (V) erhältlich sind: 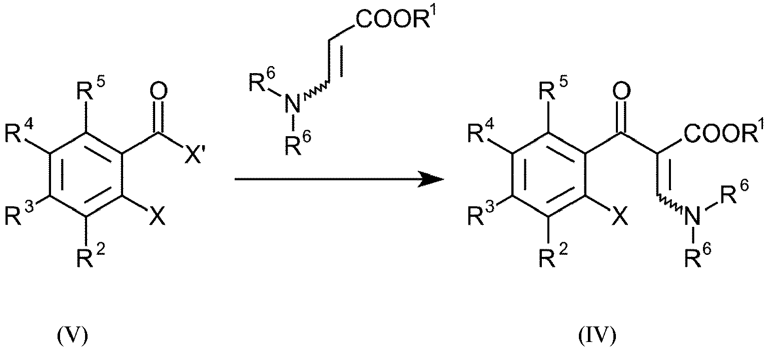 und wobei R¹ bis R⁶, X und Y die vorgenannten Bedeutungen haben und X' für Halogen steht.

## Claims

1. A method for the hydrolysis of quinolone carboxylic esters of the general formula (II) to obtain quinolone carboxylic acids of the general formula (I): 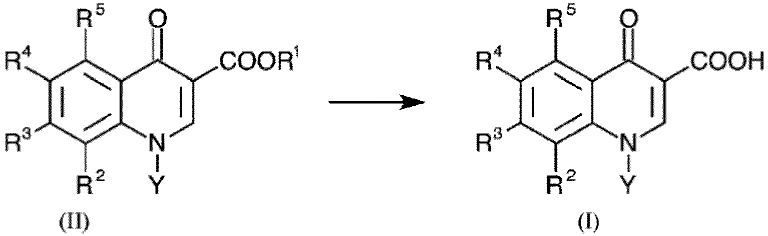
wherein, in formulae (I) and (II) concordantly,
R² is hydrogen, methyl, methoxy, fluorine, chlorine, nitro or cyano,
R³ is fluorine or chlorine,
R⁴ is fluorine,
R⁵ is hydrogen, methyl, fluorine, chlorine or nitro,
Y is methyl, ethyl, isopropyl, cyclopropyl, fluorocyclopropyl, 4-fluorophenyl or 2,4-difluorophenyl
and, in formula (I), R¹ is methyl or ethyl,
comprising the step of:
A) reacting compounds of formula (II) with a mixture comprising acetic acid, sulfuric acid and water
**characterized in that,**
in step A), ≥ 30 to ≤ 40 mol of acetic acid, ≥ 0.3 to ≤ 1 mol of sulfuric acid and ≥ 0.9 to ≤ 2.5 mol of water are used per mole of compounds of the formula (II).

2. The method according to claim 1, wherein, in step A), ≥ 95 mol% of the compounds of formula (II) that are used are converted to compounds of formula (I).

3. The method according to claim 1 or 2, wherein the reaction in step A) is carried out at a temperature of ≥ 90 to ≤ 99°C.

4. The method according to any one of claims 1 to 3, wherein formula (I) is 1-cyclopropyl-6,7,8-trifluoro-1,4-dihydro-4-oxo-3-quinoline-carboxylic acid, 1-cyclopropyl-6,7-difluoro-1,4-dihydro-4-oxo-3-quinoline-carboxylic acid, 1-cyclopropyl-6,7-difluoro-8-cyano-1,4-dihydro-4-oxo-3-quinoline-carboxylic acid, 1-(2-fluoro)cyclopropyl-6,7-difluoro-1,4-dihydro-4-oxo-3-quinoline-carboxylic acid, 1-cyclopropyl-8-chloro-6,7-difluoro-1,4-dihydro-4-oxo-3-quinoline-carboxylic acid, 1-ethyl-6,7,8-trifluoro-1,4-dihydro-4-oxo-3-quinoline-carboxylic acid or 1-cyclopropyl-6-fluoro-7-chloro-8-cyano-1,4-dihydro-4-oxo-3-quinoline-carboxylic acid.

5. The method according to any one of claims 1 to 4, wherein formulae (II) and (I) have the following meaning according to formulae (II-1) or (I-1):

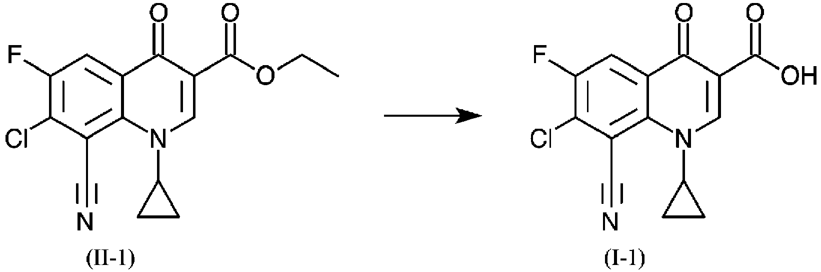

6. The method according to any one of claims 1 to 5, wherein the compounds of formula (II) can be obtained by reacting compounds of the general formula (III): 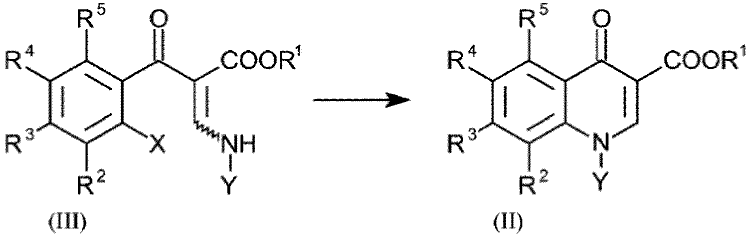 wherein R¹ to R⁵ and Y have the above meanings and X is halogen.

7. The method according to claim 6, wherein the compounds of formula (III) can be obtained by reacting compounds of the general formula (IV): 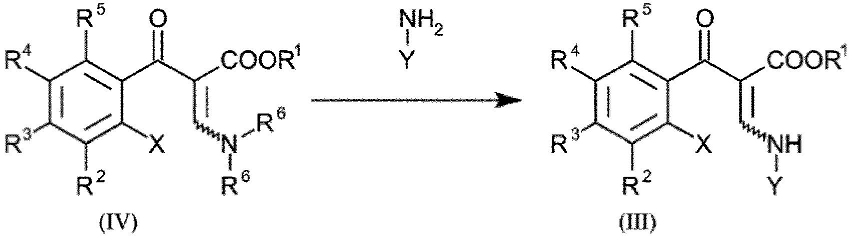 and wherein R¹ to R⁵, X and Y each have the above meanings and R⁶ is C₁-C₄ alkyl.

8. The method according to claim 7, wherein the compounds of formula (IV) can be obtained by reacting compounds of the general formula (V): 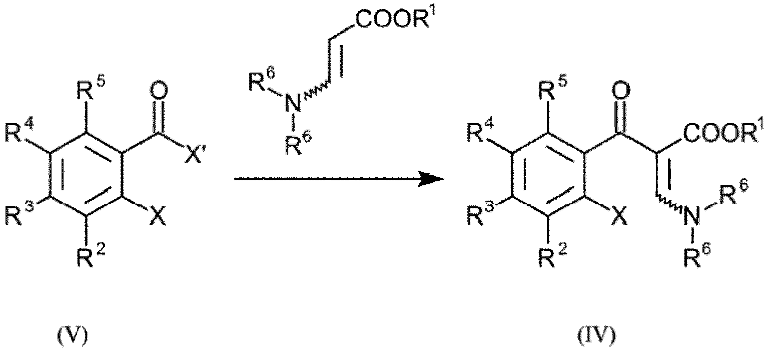 and wherein R¹ to R⁶, X and Y have the above meanings and X¹ is halogen.

## Revendications

1. Procédé d'hydrolyse d'esters d'acides quinolone-carboxyliques de formule générale (II) avec obtention d'acides quinolone-carboxyliques de formule générale (I) : 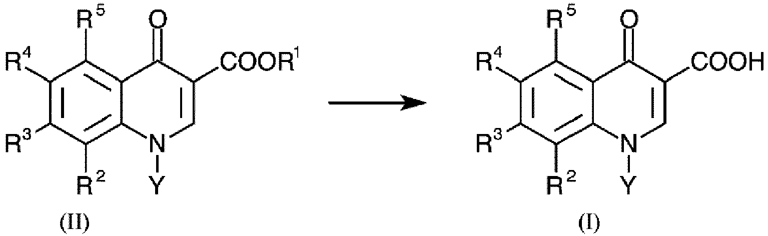
où, dans les formules (I) et (II), de manière concordante
R² représente hydrogène, méthyle, méthoxy, fluor, chlore, nitro ou cyano,
R³ représente fluor ou chlore,
R⁴ représente fluor,
R⁵ représente hydrogène, méthyle, fluor, chlore ou nitro,
Y représente méthyle, éthyle, isopropyle, cyclopropyle, fluorocyclopropyle, 4-fluorophényle ou 2,4-difluorophényle
et, dans la formule (I), R¹ représente méthyle ou éthyle,
comprenant l'étape de :
A) réaction de composés de formule (II) avec un mélange comprenant de l'acide acétique, de l'acide sulfurique et de l'eau
**caractérisé en ce que**
à l'étape A), par mole de composés de formule (II), on utilise ≥ 30 à ≤ 40 moles d'acide acétique, ≥ 0,3 à ≤ 1 mole d'acide sulfurique et ≥ 0,9 à ≤ 2,5 moles d'eau.

2. Procédé selon la revendication 1, dans lequel, à l'étape A), ≥ 95 % en moles des composés utilisés de formule (II) sont transformés en composés de formule (I).

3. Procédé selon la revendication 1 ou 2, dans lequel la réaction à l'étape A) est réalisée à une température de ≥ 90 à ≤ 99 °C.

4. Procédé selon l'une des revendications 1 à 3, dans lequel la formule (I) représente l'acide 1-cyclopropyl-6,7,8-trifluoro-1,4-dihydro-4-oxo-3-quinoléine-carboxylique, l'acide 1-cyclopropyl-6,7-difluoro-1,4-dihydro-4-oxo-3-quinoléine-carboxylique, l'acide 1-cyclopropyl-6,7-difluoro-8-cyano-1,4-dihydro-4-oxo-3-quinoléine-carboxylique, l'acide 1-(2-fluoro)cyclopropyl-6,7-difluoro-1,4-dihydro-4-oxo-3-quinoléine-carboxylique, l'acide 1-cyclopropyl-8-chloro-6,7-difluoro-1,4-dihydro-4-oxo-3-quinoléine-carboxylique, l'acide 1-éthyl-6,7,8-trifluoro-1,4-dihydro-4-oxo-3-quinoléine-carboxylique ou l'acide 1-cyclopropyl-6-fluoro-7-chloro-8-cyano-1,4-dihydro-4-oxo-3-quinoléine-carboxylique.

5. Procédé selon l'une des revendications 1 à 4, dans lequel les formules (II) et (I) présentent la signification suivante selon les formules (II-1) ou (I-1) :

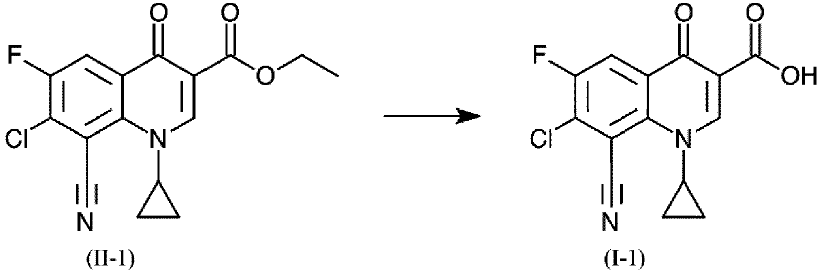

6. Procédé selon l'une des revendications 1 à 5, dans lequel les composés de formule (II) peuvent être obtenus par réaction de composés de formule générale (III) : 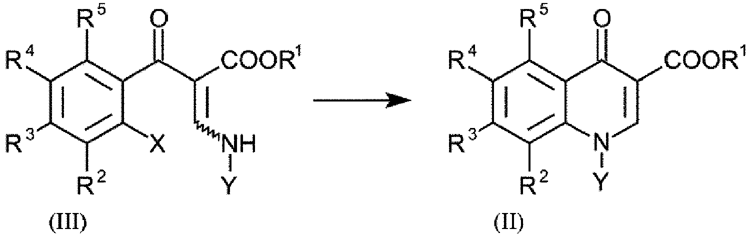 où R¹ à R⁵ et Y présentent les significations susmentionnées et X représente halogène.

7. Procédé selon la revendication 6, dans lequel les composés de formule (III) peuvent être obtenus par réaction de composés de formule générale (IV) : 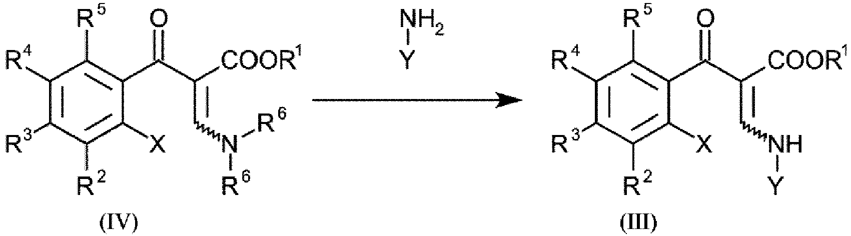 et où R¹ à R⁵, X et Y présentent les significations susmentionnées et R⁶ représente un alkyle en C₁-C₄.

8. Procédé selon la revendication 7, dans lequel les composés de formule (IV) peuvent être obtenus par réaction de composés de formule générale (V) : 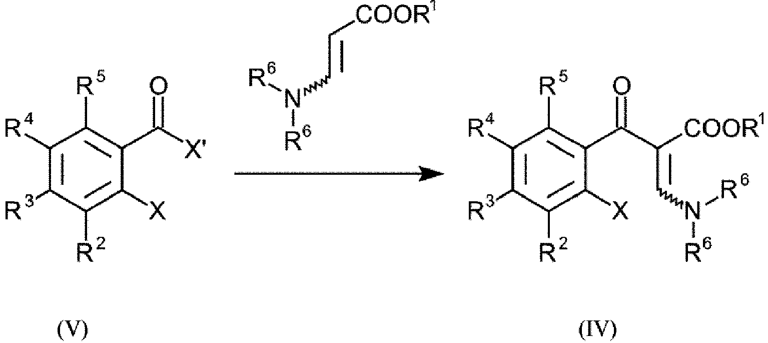 et où R¹ à R⁶, X et Y présentent les significations susmentionnées et X' représente halogène.
